Europäisches Patentamt

(19) European Patent Office

Office européen des brevets

(11) Numéro de publication : **0 458 714 A1**

(12) # DEMANDE DE BREVET EUROPEEN

(21) Numéro de dépôt : **91470009.1**

(22) Date de dépôt : **14.05.91**

(51) Int. Cl.$^5$ : **G01N 33/20, G01N 3/46**

(30) Priorité : **17.05.90 FR 9006168**

(43) Date de publication de la demande :
**27.11.91 Bulletin 91/48**

(84) Etats contractants désignés :
**AT BE CH DE DK ES GB IT LI LU NL SE**

(71) Demandeur : **INSTITUT DE RECHERCHES DE LA SIDERURGIE FRANCAISE (IRSID) Immeuble Elysées-la-Défense 19, Le Parvis F-92800 Puteaux La Défence 4 (FR)**

(72) Inventeur : **Kaplan, Dominique 16, rue Pauline Kreuscher F-78600 Maisons-Laffite (FR)**

(74) Mandataire : **Ventavoli, Roger et al TECHMETAL PROMOTION Domaine de l'IRSID Voie romaine BP 321 F-57213 Maizières-lès-Metz Cédex (FR)**

(54) **Procédé et dispositif de détermination de la teneur locale en carbone d'un produit en acier.**

(57)   On réalise un échauffement localisé de l'acier dans la zone de mesure (5) suivi d'un refroidissement, de manière à produire une trempe martensitique superficielle de l'acier sans refusion et avec une diffusion limitée de carbone. On mesure la dureté de l'acier dans la zone (5) trempée superficiellement et on déduit la teneur locale en carbone de la valeur mesurée de la dureté superficielle. L'échauffement localisé de l'acier dans la zone (5) est réalisé de préférence par balayage par un faisceau laser (3).

FIG.1

EP 0 458 714 A1

L'invention concerne un procédé et un dispositif de détermination de la teneur locale en carbone d'un produit en acier, tel qu'une tôle forte obtenue par coulée continue.

La teneur locale en carbone de produits en acier peut avoir une influence importante sur certaines propriétés de l'acier telles que sa ténacité ou sa résistance à la corrosion dans certains milieux. Il est donc important de pouvoir estimer la teneur locale en carbone de l'acier ou la répartition du carbone, dans certaines zones situées sur une surface accessible du produit, ou d'un échantillon de celui-ci. Par exemple, dans le cas de tôles fortes, il peut être nécessaire de connaître les variations de la teneur en carbone en fonction de la profondeur à partir de la surface de la tôle, dans une direction correspondant à son épaisseur.

Pour effectuer une telle détermination ou évaluation, on utilise actuellement des méthodes telles que l'analyse par microsonde électronique ou l'analyse spectroscopique. Ces méthodes sont d'une mise en oeuvre complexe et nécessitent l'utilisation de dispositifs coûteux et encombrants qui se prêtent mal à une utilisation dans une usine sidérurgique, à la sortie d'une ligne de fabrication de produits en continu.

Le but de l'invention est donc de proposer un procédé de détermination de la teneur locale en carbone d'un produit en acier, dans au moins une zone de mesure située sur une surface du produit, ce procédé pouvant être mis en oeuvre de manière simple et rapide sans nécessiter l'utilisation de dispositif d'analyse tel qu'une microsonde électronique.

Dans ce but :
– on réalise un échauffement localisé de l'acier dans la zone de mesure suivi d'un refroidissement, de manière à produire une trempe martensitique superficielle de l'acier sans refusion et avec une diffusion limitée de carbone,
– on mesure la dureté de l'acier dans la zone trempée superficiellement,
– et on deduit la teneur locale en carbone de l'acier dans la zone trempée, de la valeur mesurée de la dureté superficielle.

L'invention est également relative à un dispositif de détermination de la teneur locale en carbone d'un produit en acier.

Afin de bien faire comprendre l'invention, on va maintenant décrire, à titre d'exemple non limitatif, en se référant aux figures jointes en annexe, un mode de réalisation du procédé et du dispositif suivant l'invention utilisés pour déterminer la répartition du carbone dans une tôle forte obtenue par coulée continue, suivant l'épaisseur de la tôle forte.

La figure 1 est une vue en perspective d'une partie d'une tôle forte en acier, sur le bord de laquelle on réalise un échauffement localisé par faisceau laser, pour la mise en oeuvre du procédé suivant l'invention.

La figure 2 est un diagramme donnant la dureté maximale d'un acier trempé, en fonction de sa teneur en carbone pour des teneurs en carbone faibles.

La figure 3 est un diagramme identique au diagramme de la figure 2, pour un intervalle de teneurs en carbone plus étendu.

La figure 4 est un diagramme donnant la répartition de la durete déterminée par rayure sur le bord d'un échantillon d'une tôle forte produite par coulée continue et ayant subi une trempe martensitique localisée dans une zone linéaire dirigée suivant son épaisseur.

La figure 5 est un diagramme analogue au diagramme de la figure 4, pour une tôle forte obtenue par coulée continue avec un pincement et une réduction en sortie de coulée inférieurs aux pincement et à la réduction de la tôle forte dont le diagramme de la dureté suivant son épaisseur est représente sur la figure 4.

La figure 6 est une vue en élévation latérale d'un dispositif de détermination de la teneur locale en carbone d'un acier, suivant l'invention.

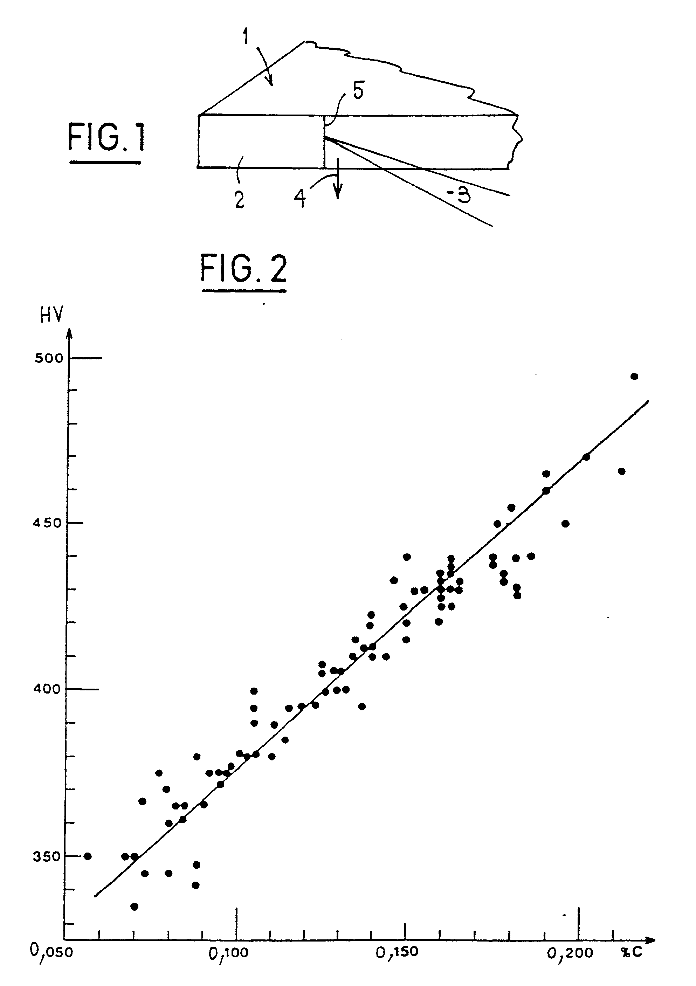

Sur la figure 1, on voit une partie d'un échantillon d'une tôle forte 1 au voisinage d'un bord 2 de cet échantillon sur lequel on réalise la détermination de la répartition de la teneur en carbone suivant l'épaisseur de la tôle, par le procédé suivant l'invention.

Pour mettre en oeuvre le procédé suivant l'invention, on réalise tout d'abord un polissage d'une zone du bord 2 de l'échantillon dans laquelle sera effectuée la trempe martensitique de l'acier et la mesure de dureté. Cette zone qui peut être de très faible largeur s'étend sur toute la hauteur du bord 2, de manière à intéresser l'ensemble de l'épaisseur de la tôle.

De manière à réaliser la trempe martensitique de l'acier dans une zone de mesure linéaire s'étendant sur toute l'épaisseur de la tôle, on soumet le bord 2 de la tôle à l'action d'un faisceau laser 3 qui est déplacé à une vitesse contrôlée dans la direction de l'épaisseur de la tôle (flèche 4), dans la zone où l'on a réalisé le polissage.

Le faisceau laser réalise un échauffement de l'acier dans une zone 5 de très faible largeur, l'échauffement très rapide produit par le faisceau laser étant suivi d'un refroidissement brusque de l'acier, lors du balayage du bord de la tôle par le faisceau laser.

Les paramètres du tir laser, c'est-à-dire la puissance et la vitesse de déplacement du faisceau sont choisis de manière à satisfaire les conditions suivantes :

1°- On réalise une trempe superficielle de l'acier de type martensitique, dans la zone de mesure balayée par le faisceau laser.

2° - On évite de provoquer une refusion partielle de l'échantillon dans la zone balayée.

3°- On évite des conditions thermiques conduisant à une diffusion notable du carbone dans la zone balayée et à son voisinage.

La première condition définit une énergie mini-

male $E_{min}$ et les deuxième et troisième conditions définissent une énergie maximale $E_{max}$. L'énergie E du faisceau laser doit donc être choisie telle que :
$E_{min} < E < E_{max}$.

Les valeurs précises des bornes $E_{min}$ et $E_{max}$ dépendent de la composition de l'acier et de la réflectivité de la surface sur laquelle on réalise le balayage.

Dans le cas d'un acier de construction E 355 à 0,15 % de carbone et 1,5 % de manganèse, on a utilisé un faisceau laser ayant une puissance de 2,4 kW qui est déplacé à une vitesse de 72 cm/mn au contact de la surface de l'acier, dans une zone ayant subi un polissage préalable.

A l'issue du balayage du bord 2 de l'échantillon par le faisceau laser 3, on obtient une zone de mesure 5 de faible largeur s'étendant sur toute l'épaisseur de la tôle et se présentant sous la forme d'une mince trace rectiligne. Dans cette zone de mesure 5, l'acier présente en surface, sur une très faible épaisseur, une structure martensitique.

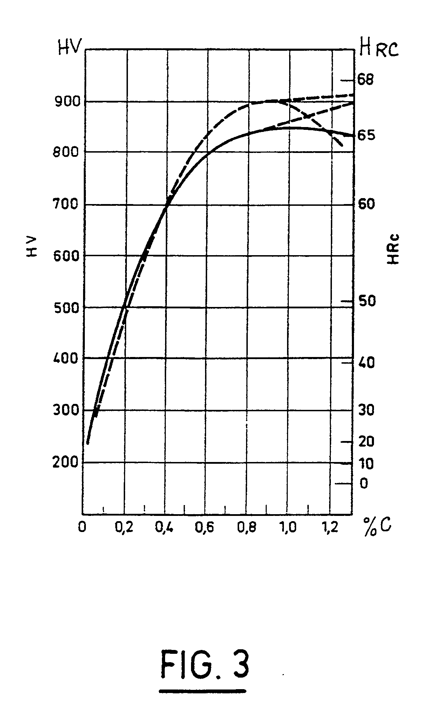

Comme il est visible sur les figures 2 et 3, la dureté d'un acier à l'état martensitique est fonction de sa teneur en carbone.

Comme il est visible sur la figure 2, pour les teneurs en carbone faibles, par exemple comprises entre 0,05 et 0,2 %, il existe une relation univoque et linéaire entre la dureté et la teneur en carbone d'un acier à l'état martensitique.

Sur la figure 2, on a porté la dureté Vickers HV de différents aciers trempés à l'état martensitique, en fonction de leur teneur en carbone, cette teneur étant comprise entre 0,05 et 0,2 % environ.

Les différents points obtenus sont légèrement dispersés autour d'une droite dont l'équation est la suivante :
HV (martensite) = 283 + 930 % C.

On en déduit :
% C = [HV (martensite) -283]/930.

On peut donc déduire de la dureté d'un acier à l'état martensitique, sa teneur en carbone de manière non ambiguë, lorsque cet acier présente une teneur en carbone inférieure à 0,2 %.

Dans le cas d'acier ayant une teneur en carbone supérieure, on pourra se reporter aux courbes de la figure 3 donnant la dureté Vickers et la dureté Rockwell d'aciers trempés à l'état martensitique en fonction de leur teneur en carbone.

En fait, les courbes représentées sur la figure 3 sont utilisables pour des aciers ayant une teneur en carbone pouvant aller jusqu'à 0,8 %. Dans le cas d'aciers ayant une teneur en carbone très forte, supérieure à 0,8 %, on ne peut déterminer de manière certaine la teneur en carbone de l'acier à partir de sa dureté, la relation entre la dureté et le pourcentage en carbone n'étant plus univoque.

Dans le cas d'un acier ayant subi une trempe superficielle de type martensitique dans une zone de mesure, il est possible de déterminer la teneur locale en carbone dans la zone de mesure, en un ou plusieurs points, en effectuant une ou plusieurs mesures de dureté superficielle dans la zone de mesure ayant subi la trempe martensitique superficielle.

A l'aide de la relation donnée ci-dessus ou en utilisant les diagrammes tels que représentés sur les figures 2 et 3, on peut déterminer la teneur locale en carbone de l'acier, à partir de sa dureté.

La ou les mesures de dureté locale dans la zone de mesure peuvent être réalisées de manière classique par points, en réalisant une empreinte sous une charge déterminée et en mesurant les dimensions de cette empreinte. La charge doit être suffisamment faible pour que l'empreinte de dureté reste à l'intérieur de la zone de mesure dans laquelle l'acier est trempé à l'état martensitique, cette zone de mesure présentant une faible largeur et une faible épaisseur.

Il est également possible et avantageux de mesurer la dureté de l'acier dans la zone de mesure, de manière continue, en réalisant une rayure sur la surface de l'acier, sous une charge constante, la profondeur de la rayure étant mesurée en continu et enregistrée. Un appareil de mesure de dureté par rayure est décrit dans la demande de brevet français FR-A-2.623.285 déposée au nom de CREUSOT-LOIRE INDUSTRIE.

Dans le cas d'une tôle forte, par exemple en acier E 355, obtenue par coulée continue, on a utilisé la méthode de mesure de dureté par rayure, pour déterminer la répartition de la teneur en carbone de l'acier, suivant l'épaisseur de la tôle.

La mesure par rayure est effectuée suivant la trace rectiligne 5 réalisée par le faisceau laser, comme décrit ci-dessus.

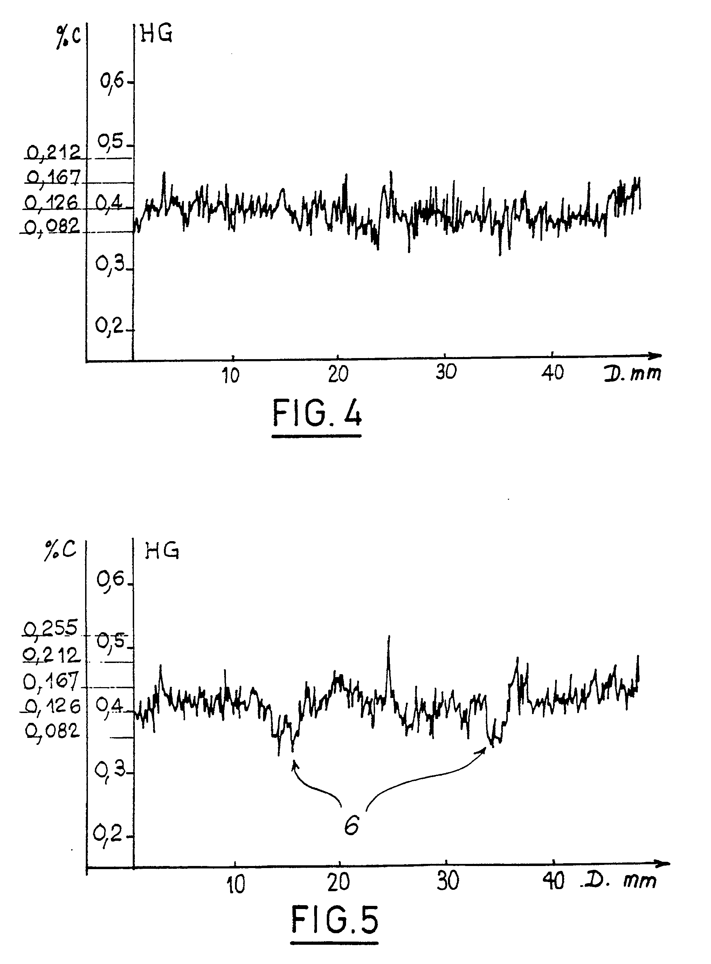

Sur les figures 4 et 5, on a représenté les variations de la dureté mesurée par rayure designée par HG, c'est-à-dire de la profondeur de la rayure réalisée sous charge constante, en fonction de la distance D en millimètre à l'une des faces de la tôle.

On a également donné sur les figures 4 et 5, les valeurs de la teneur locale en carbone correspondant aux duretés mesurées par rayure, dans le sens de l'épaisseur de la tôle.

Les mesures ont été effectuées sur deux tôles d'épaisseur 50 mm, en acier E355 à 0,145 % C, ayant des analyses sensiblement identiques et obtenues par coulée continue.

Les deux tôles sur lesquelles ont été réalisées les essais dont les résultats sont représentés sur les figures 4 et 5 diffèrent par les conditions de pincement en sortie de coulée continue.

En effet, après coulée et refroidissement du produit plat destiné à constituer la tôle forte, ce produit plat subit une réduction d'épaisseur, pour l'amener à son épaisseur définitive, entre des cylindres analogues à des cylindres de laminoir. Pendant cette opération de réduction d'épaisseur appelée "réduction douce", le produit plat est totalement solidifié en surface et présente une consistance pâteuse à coeur. La réduction d'épaisseur du produit se traduit par une compression de sa partie centrale qui influe sur la répartition des éléments de l'acier et sur la formation de bandes de ségrégation dans le produit plat à l'état totalement solidifié.

Il est possible, pour une épaisseur donnée du produit plat à l'état fini, d'effectuer une réduction douce, avec un taux de réduction plus ou moins important.

On a utilise le procédé suivant l'invention, pour déterminer la répartition de la teneur en carbone, suivant l'épaisseur d'une première tôle forte ayant subi une forte réduction douce (figure 4) et d'une seconde tôle ayant subi une faible réduction douce (figure 5), les deux tôles ayant par ailleurs la même composition et la même épaisseur de 50 mm.

En comparant les figures 4 et 5, on peut remarquer que la tôle ayant subi le pincement le plus important et la réduction la plus forte présente une plus grande homogénéité et des teneurs en carbone locales extrêmes moins divergentes que la tôle ayant subi le plus faible pincement et la plus faible réduction.

La tôle ayant subi la plus faible réduction (figure 5) présente en particulier des zones, correspondant aux zones 6 de la courbe, dans lesquelles la teneur en carbone est inférieure à la teneur moyenne, de part et d'autre de son axe.

Les mesures de dureté par rayure sur le bord des tôles, suivant la zone durcie par trempe martensitique ont été réalisées sous une charge constante de 2,5 N. Avant d'effectuer les mesures de dureté, on vérifie de manière visuelle qu'il n'y a pas eu de refusion dans la zone trempée. Les courbes obtenues représentant les variations de profondeur de la rayure suivant la longueur de la zone de mesure, dans le sens de l'épaisseur du produit, sont représentatives des variations de la dureté locale superficielle de l'acier trempé et donc de la teneur locale en carbone de cet acier.

On a reporté sur les figures 4 et 5 les valeurs de la teneur locale en carbone de l'acier.

Ces teneurs correspondent effectivement aux teneurs locales en carbone de la zone correspondante de la tôle, dans la mesure où l'échauffement par le faisceau laser a été réalisé de manière à éviter une diffusion notable du carbone dans la zone échauffée et une refusion de l'acier.

Les teneurs en carbone se repartissent autour d'une valeur moyenne de l'ordre de 0,145 %, ce qui correspond à la teneur moyenne en carbone de l'acier E 355. Ceci permet de vérifier a posteriori le caractère significatif des mesures effectuées.

Les teneurs locales en carbone présentent des valeurs extrêmes correspondant à des ségrégations positives ou négatives dans certaines zones de la tôle.

La dispersion est nettement plus faible dans le cas de la tôle ayant subi un plus fort pincement.

On a effectué un examen microscopique classique du bord des tôles, au voisinage de la zone de mesure, après polissage et attaque au nital de l'acier. L'examen microscopique confirme la présence de bandes ségrégées ayant une structure bainito-martensitique nettement plus affirmée dans le cas de la tôle forte dont les résultats de mesure sont représentés sur le diagramme de la figure 5.

On a également effectué des essais de résilience à -40°C sur des éprouvettes prélevées dans les tôles fortes. Ces essais montrent que le pourcentage d'éprouvettes ayant présenté une énergie de rupture inférieure ou égale à 40 Joules est de 27 % dans le cas de la tôle forte ayant subi le plus fort pincement et de 68 % dans le cas de la tôle forte ayant subi le plus faible pincement.

Ces essais mettent clairement en évidence le caractère néfaste des bandes ségrégées dont la teneur locale en carbone est forte, sur la résilience de l'acier.

Ces résultats sont parfaitement en accord avec les profils obtenus par le procédé de l'invention et représentés sur les figures 4 et 5 et montrent l'intérêt particulier du procédé selon l'invention pour le contrôle de la ségrégation interne de produits sidérurgiques tels que les tôles épaisses.

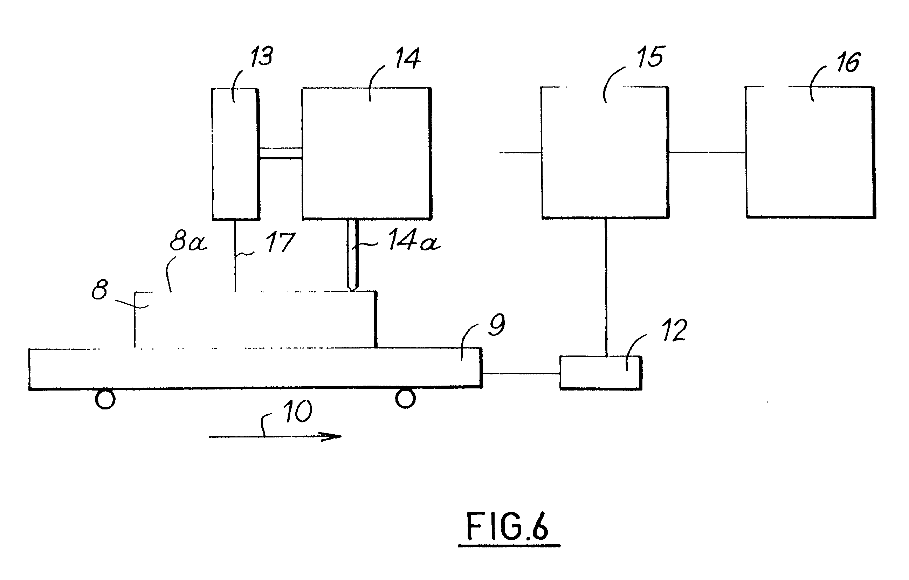

Sur la figure 6, on a représente de manière schématique un dispositif permettant de mettre en oeuvre le procédé suivant l'invention de manière simple et rapide, avec une visualisation directe de la répartition de la teneur locale en carbone sur une surface d'un échantillon d'un produit en acier.

L'échantillon 8 est fixé sur une table 9 mobile dans la direction de la flèche 10, grâce à un moyen de déplacement 12 tel qu'un moteur associe à un ensemble pignon et crémaillère ou un vérin.

Au-dessus de la surface 8a de l'échantillon 8 sur laquelle on effectue la détermination de la teneur locale en carbone, sont disposés successivement dans le sens donné par la flèche 10, un générateur de faisceau laser 13 et un appareil de mesure de dureté par rayure 14.

L'appareil de mesure de dureté 14 et la table 9 de déplacement de l'échantillon peuvent être réalisés comme décrit dans le FR-A-2.623.285.

Les signaux des capteurs de mesure de l'appareil 14 sont transmis à un calculateur 15 relié à un dispositif d'affichage 16 tel qu'un traceur de courbe ou un écran de visualisation.

Pendant le fonctionnement du dispositif, l'échantillon est déplacé dans la direction et le sens de la flèche 10. Le faisceau laser 17 réalise un échauffement rapide d'une zone étroite et rectiligne de la surface 8a de l'échantillon 8, suivi d'un refroidissement dans des conditions telles que l'échantillon 8 subit une trempe martensitique superficielle.

Une mesure de dureté par rayure est effectuée de manière continue par l'appareil de mesure 14 dont la pointe de mesure 14a située dans l'alignement du faisceau laser 17 suit la zone étroite et rectiligne dans laquelle a été réalisée la trempe martensitique.

Les mesures de dureté par rayure sont traitées par le calculateur 15 et affichées directement sur l'écran 16 sous la forme d'une courbe donnant la répartition de la teneur locale en carbone suivant la zone de l'échantillon balayée par le faisceau laser.

Il est bien évident que le dispositif pourrait être conçu de manière à permettre un déplacement simultané du générateur de faisceau laser et de l'appareil de mesure de dureté au-dessus de la surface 8a de l'échantillon 8 en position fixe.

Il est à remarquer que le procédé de détermination suivant l'invention peut être un procédé non destructif qui peut être mis en oeuvre de manière rapide sur une surface du produit métallurgique, par exemple sur un bord de ce produit dans le cas d'une tôle, la mise en oeuvre du procédé suivant l'invention ne nécessitant pas alors de réaliser un découpage ou une altération du produit, l'effet local d'échauffement et de trempe dans la zone de mesure ayant un effet tout-à-fait négligeable.

L'invention ne se limite pas au mode de réalisation qui a été décrit.

C'est ainsi qu'on peut réaliser l'échauffement et la trempe superficielle du produit en acier, par un moyen différent d'un faisceau laser et, par exemple, en utilisant un faisceau d'électrons ou un dispositif de chauffage par induction, suivant la forme et l'étendue de la zone de mesure et les caractéristiques de trempe de l'acier.

Cependant, le faisceau laser est très nettement préférable en raison du faible temps d'interaction entre le faisceau et le métal, qui minimise la diffusion du carbone.

Il est en effet nécessaire d'éviter cette diffusion pour garantir l'exactitude de la teneur locale en carbone. On est donc conduit à limiter le plus possible le temps d'interaction entre le faisceau laser et le métal et la profondeur traitée (de l'ordre de 0,10 mm). Il est à noter que la technique de mesure de dureté par rayure s'accommode très bien de cette faible profondeur traitée, alors que d'autres techniques de mesure de dureté nécessitent une profondeur plus importante donc un traitement laser plus accentué et donc un risque accru de diffusion du carbone.

La zone de mesure peut être constituée par une bande ou une ligne continue ou encore par un ou plusieurs points isolés.

La mesure de dureté peut être réalisée par tout moyen permettant d'obtenir avec une bonne précision, la valeur de la dureté superficielle locale, dans la zone ayant subi la trempe martensitique.

Enfin, le procédé suivant l'invention s'applique dans le cas de tout produit en acier obtenu par un procédé quelconque (coulée continue, moulage, laminage ou filage).

## Revendications

1.- Procédé de détermination de la teneur locale en carbone d'un produit en acier, dans au moins une zone de mesure (5) située sur une surface (2) du produit (1), caractérisé par le fait :
- qu'on réalise un échauffement localisé de l'acier dans la zone de mesure (5) suivi d'un refroidissement, de manière à produire une trempe martensitique superficielle de l'acier sans refusion et avec une diffusion limitée de carbone,
- qu'on mesure la dureté de l'acier dans la zone (5) trempée superficiellement,
- et qu'on déduit la teneur locale en carbone de l'acier dans la zone trempée (5), de la valeur mesurée de la dureté superficielle.

2.- Procédé suivant la revendication 1, caractérisé par le fait qu'on réalise l'échauffement localisé de l'acier dans la zone de mesure (5) en utilisant un faisceau laser (3).

3.- Procédé suivant la revendication 2, dans le cas d'un acier de construction à 0,15 % de carbone et 1,5 % de manganèse, caractérisé par le fait que le faisceau laser a une puissance voisine de 2,4 kW et qu'on balaie la zone de mesure (5) avec le faisceau laser à une vitesse voisine de 72 cm/mn.

4.- Procédé suivant l'une quelconque des revendications 1 à 3, caractérisé par le fait que la zone de mesure (5) est constituée par une trace rectiligne de faible largeur sur la surface de l'acier.

5. - Procédé suivant l'une quelconque des revendications 1 à 4, caractérisé par le fait qu'on mesure la dureté de l'acier dans la zone (5) trempée superficiellement, par points, en réalisant une ou plusieurs empreintes dont on mesure les dimensions.

6. - Procédé suivant l'une quelconque des revendications 1 à 4, caractérisé par le fait qu'on réalise la mesure de dureté dans la zone (5) trempée superficiellement, par rayure.

7.- Procédé suivant l'une quelconque des revendications 1 à 6, dans le cas d'un acier ayant une teneur moyenne en carbone inférieure à 0,2 %, caractérisé par le fait qu'on déduit la teneur locale en carbone de l'acier de la valeur mesurée de la dureté superficielle, à partir d'une relation linéaire prédéterminée.

8.- Dispositif de détermination de la teneur locale en carbone d'un produit (8) en acier, caractérisé en ce qu'il comprend des moyens de chauffage (13), pour réaliser un échauffement rapide et localisé de la surface du produit, liés à des moyens de mesure (14) de la dureté de la surface, et des moyens de déplacement (9) pour déplacer ledit produit (8) relativement auxdits moyens de chauffage (13) et de mesure de dureté (14).

9.- Dispositif selon la revendication 8, caractérisé en ce que les moyens de chauffage (13) sont constitués par une source de rayon laser.

**10.-** Dispositif selon la revendication 8, caractérisé en ce que les moyens de mesure (14) de la dureté sont constitués par un dispositif de mesure de la dureté par rayure.

## FIG.1

## FIG.2

FIG. 3

FIG. 4

FIG.5

FIG.6

EP 0 458 714 A1

Office européen
des brevets

**RAPPORT DE RECHERCHE EUROPEENNE**

Numero de la demande

EP  91 47 0009

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | ASM HEAT TREATING DIVISION COUNCIL: "METALS HANDBOOK" 9ème édition, vol. 4, pages 507-517, 1985, American Society for Metals, Ohio, US * document en entier * --- | 1-10 | G 01 N   33/20 G 01 N    3/46 |
| Y | W.T. LANKFORD, JR et al.: "THE MAKING, SHAPING AND TREATING OF STEEL" 10ème édition, pages 1241-1249,1408-1416, 1985, Herbick & Held, Pittsburgh, PA, US * figures26-41; page 1414, colonne de droite * --- | 1-10 | |
| A | ASM HANDBOOK COMMITEE: "METALS HANDBOOK" 9ème édition, vol. 1, pages 471-476, 1978, American Society for Metals, Ohio, US * figure 1 * --- | 1,7 | |
| A | US-A-4 719 583  (H. TAKAFUJI et al.) * figure 7; colonne 8, lignes 40-43 * ----- | 8 | **DOMAINES TECHNIQUES RECHERCHES (Int. Cl.5)** G 01 N |

Le présent rapport a été établi pour toutes les revendications

| Lieu de la recherche | Date d'achèvement de la recherche | Examinateur |
|---|---|---|
| BERLIN | 27-08-1991 | BRISON O.P. |